# EUROPEAN PATENT SPECIFICATION

(11) **EP 1 131 096 B1**
(45) Date of publication and mention of the grant of the patent: **06.01.2010**
(21) Application number: 99958649.8
(22) Date of filing: 09.11.1999
(51) Int. Cl.: A61K 39/395, A61K 39/00, A61K 35/02

(54) **ANTI-CD20 ANTIBODY TREATMENT OF PATIENTS RECEIVING BMT OR PBSC TRANSPLANTS**
BEHANDLUNG VON PATIENTEN DIE EINE KNOCHENMARKTRANSPLANTATION ODER EINE TRANSPLANTATION PERIPHERER BLUTSTAMMZELLEN ERHALTEN MIT ANTI-CD20 ANTIKÖRPERN
TRAITEMENT PAR ANTICORPS ANTI-CD20, DE PATIENTS RECEVEURS DE GREFFES DE MOELLE OSSEUSE OU DE CELLULES SOUCHES DE SANG PERIPHERIQUE

(30) Priority: 09.11.1998 US 107657 P
(43) Date of publication of application: 12.09.2001
(73) Proprietor: Biogen Idec Inc., Cambridge, MA 02142 (US)
(72) Inventor: GRILLO-LOPEZ, Antonio, J., Rancho Santa Fe, CA 92067 (US); LEONARD, John, E., Carlsbad, CA 92009 (US)
(74) Representative: Walton, Seán Malcolm
(86) International application number: PCT/US1999/024012
(87) International publication number: WO 2000/027433

(56) References cited:
- US-A- 5 736 137
- MCLAUGHLIN P ET AL: "Rituximab chimeric anti-CD20 monoclonal antibody therapy for relapsed indolent lymphoma: half of patients respond to a four-dose treatment program." JOURNAL OF CLINICAL ONCOLOGY, (1998 AUG) 16 (8) 2825-33. , XP001027531
- JANAKIRAMAN, N. (1) ET AL: "Rituximab: Correlation between effector cells and clinical activity in NHL." BLOOD, (NOV. 15, 1998) VOL. 92, NO. 10 SUPPL. 1 PART 1-2, PP. 337A. MEETING INFO.: 40TH ANNUAL MEETING OF THE AMERICAN SOCIETY OF HEMATOLOGY MIAMI BEACH, FLORIDA, USA DECEMBER 4-8, 1998 THE AMERICAN SOCIETY OF HEAMATOLOGY. , XP001009992
- MCLAUGHLIN, P. (1) ET AL: "Efficacy controls and long-term follow-up of patients (PTS) treated with rituximab for relapsed or refractory, low-grade or follicular (R-LG/F) NHL." BLOOD, (NOV. 15, 1998) VOL. 92, NO. 10 SUPPL. 1 PART 1-2, PP. 414A -415A. MEETING INFO.: 40TH ANNUAL MEETING OF THE AMERICAN SOCIETY OF HEMATOLOGY MIAMI BEACH, FLORIDA, USA DECEMBER 4-8, 1998 THE AMERICAN SOCIETY OF HEAMATOLOGY. , XP000952700
- ROTTENBURGER, CHRISTOF ET AL: "Clonotypic CD20+ and CD19+ B cells in peripheral blood of patients with multiple myeloma post high-dose therapy and peripheral blood stem cell transplantation" BR. J. HAEMATOL. (1999), 106(2), 545-552 , XP001038308
- FLINN ET AL BIOL BLOOD MARROW TRANSPL vol. 6, 2000, pages 628 - 632
- KHOURI ET AL BLOOD vol. 98, no. 13, 2001, pages 3595 - 3599
- MANGEL ET AL SEMINARS IN ONCOLOGY vol. 29, no. 1, 2002, pages 56 - 69
- BIERMAN ET AL: 'High-dose therapy with autologous hematopoietic rescue for follicular low-grade non-Hodgkin's lymphoma' JOURNAL OF CLINICAL ONCOLOGY vol. 15, no. 2, 1997, pages 445 - 450

## Description

### FIELD OF THE INVENTION

The use of an anti-CD20 antibody or a fragment thereof as an *in vivo* purging agent in patients receiving bone marrow transplant or peripheral blood stem cell transplant is disclosed.

### BACKGROUND OF THE INVENTION

The use of antibodies to the CD20 antigen as diagnostic and/or therapeutic agents for B-cell lymphoma has previously been reported. CD20 is a useful marker or target for B-cell lymphomas as this antigen is expressed at very high densities on the surface of malignant B-cells, i.e., B-cells wherein unabated proliferation can lead to B-cell lymphomas.

CD20 or Bp35 is a B-lymphocyte-restricted differentiation antigen that is expressed during early pre-B-cell development and remains until plasma cell differentiation. It is believed by some that the CD20 molecule may regulate a step in the B-cell activation process which is required for cell cycle initiation and differentiation. Moreover, as noted, CD20 is usually expressed at very high levels on neoplastic ("tumor") B-cells.

Previous reported therapies involving anti-CD20 antibodies have involved the administration of a therapeutic anti-CD20 antibody either alone or in conjunction with a second radiolabeled anti-CD20 antibody, or a chemotherapeutic agent.

In fact, the Food and Drug Administration has approved the therapeutic use of one such therapeutic anti-CD20 antibody, RITUXAN®, for use in relapsed and previously treated low-grade non-Hodgkin's lymphoma (NHL).

Also, the use of RITUXAN® in combination with a radiolabeled murine anti-CD20 antibody has been suggested for the treatment of B-cell lymphoma.

However, while anti-CD20 antibodies and, in particular, RITUXAN®, have been reported to be effective for treatment of B-cell lymphomas, such as non-Hodgkin's lymphoma, the treated patients are often subject to disease relapse. Therefore, it would be beneficial if more effective antibody treatments could be developed. More specifically, it would be advantageous if other therapeutic applications of anti-CD20 antibodies were discovered. Also, it would be helpful if current treatment protocols for B-cell lymphoma were improved, which prevented or further reduced disease relapse.

### BRIEF DESCRIPTION OF THE INVENTION

Thus, it is an object of the invention to improve the problems of prior treatments of B-cell-related diseases, e.g., B-Cell lymphomas and leukemias, in particular the problem of disease relapse after disease treatment.

More specifically, it is an object of the invention to reduce the incidence of disease relapse in patients with B-cell-related diseases receiving bone marrow or peripheral blood stem cell transplants by the use of an anti-CD20 antibody as an *in vivo* purging agent, concurrent, and/or up to 100 days after transplant.

It is an even more specific object of the invention to use RITUXAN® as an *in vivo* purging agent, concurrent and/or up to 100 days after bone marrow or peripheral blood stem cell transplant.

### DETAILED DESCRIPTION OF THE INVENTION

A significant problem associated with the treatment of diseases involving B-cells and other cells that express CD20 antigen, including B-cell lymphomas and leukemias, is the problem of disease relapse afer treatment.

The exact cause for disease relapse is unclear. However, it is known that such relapse may occur even in patients that receive aggressive therapeutic intervention, e.g., high dosages of chemotherapeutic agents, cytokines, radiation, and/or antibody. While the exact cause of relapse remains unclear, it is speculated by some researchers that disease relapse may occur because the patient may still harbor low numbers of diseased cells even after aggressive therapy. Also, it is speculated that bone marrow transplant or peripheral blood stem cell transplanted tissue may itselfbe contaminated by diseased cells that express the CD20 antigen, e.g., B-cell lymphoma cells. Therefore, transplant of such tissues may unwittingly introduce diseased cells, and thereby actually increase the risk of disease relapse.

As discussed, the present invention seeks to prevent or reduce the incidence of disease in patients receiving transplanted bone marrow or peripheral blood stem cells. The present invention provides use of an anti-CD20 antibody as claimed.

Bone marrow or peripheral blood stem cells may be contacted in tissue culture with an anti-CD20 antibody prior to transplant. In the preferred embodiment such antibody will comprise a chimeric, primate, primatized®, humanized or human anti-CD20 antibody, preferably RITUXAN®.

The patient is treated concurrent with or up to 100 days after bone marrow or peripheral blood stem cell transplant with an amount of an anti-CD20 antibody effective to purge (*in vivo*) or at least reduce the number of disease causing cells that express CD20 antigen that may be present in the transplant.

Similarly, the antibody used for *in vivo* purging will preferably comprise a chimeric, humanized, primate, primatized®, or human anti-CD20 antibody, preferably RITUXAN®. This *in vivo* purging may be effected simultaneous or substantially contemporaneous to bone marrow or peripheral blood stem cell transplant. Preferably, such purging will be effected within a week or more, preferably within 1 to 12 hours after transplant. However, such purging can be effected up to about 1 to 100 days after transplant. In the preferred embodiment, *in vivo* purging will be effected within about 1 month after transplant, more preferably within about one week after transplant, and most preferably within about 1 to 12 hours after transplant.

As noted above, the subject *in vivo* purging of CD20 antigen-expressing cells will be effected in patients that have previously been treated in an effort to eradicate disease causing B-cells, or other CD20 antigen-expressing cells involved in disease. Such treatment methods include, by way of example, cytokine therapy, antibody therapies (e.g., RITUXAN® or other antibodies targeted to B-cells), chemotherapy and/or radiation therapy, e.g.; whole body irradiation, radioimmunotherapy.

In an especially preferred embodiment, the subject *in vivo* purging will be effected in patients that have previously been treated with RITUXAN® and/or radioimmunotherapy that receive an autologous bone marrow or peripheral blood stem cell transplant after RIT and/or RITUXAN® therapy.

For example, patients that have a B-cell-related disease, e.g., a B-cell lymphoma or leukemia, will have their bone marrow or peripheral blood stem cells collected prior to therapeutic treatment. This will be effected by known methods.

The patient will then be subjected to an aggressive therapeutic regimen, e.g., administration of RITUXAN®, or a radiolabeled antibody that is specific to an antigen expressed by the tumor cells, whole body irradiation, and/or a chemotherapeutic or cytokine. This therapeutic regimen will be effected under conditions that are hypothetically designed to eradicate any B-cell or other CD20 antigen-expressing tumor cells that may be present.

After such treatment has been completed, the bone marrow or peripheral blood stem cells, which optionally may be treated *in vitro* with an anti-CD20 antibody, e.g., RITUXAN®, to deplete CD20 expressing cells, is then transplanted into the patient in order to reconstitute the immune system thereof.

Concurrently or shortly thereafter, the patient will be administered an amount of an anti-CD20 antibody, e.g., RITUXAN®, effective to purge any disease causing cells that may be present in the bone marrow or peripheral blood stem cell transplant. An effective dosage will typically comprise from about 0.01 to about 3.0 mg/kg body weight. A preferred dosage will comprise from about 1 to about 20 mg/kg, more preferably from about 1 to about 5.0 mg/kg, administered within about one week of transplant.

The subject *in vivo* purging will reduce the risk of relapse in many B-cell-related diseases, e.g., B-cell lymphomas and leukemias such as non-Hodgkin's lymphomas, chronic lymphocytic leukemia, etc., after treatment has been completed in patients receiving transplanted cells that potentially may be contaminated with disease-causing cells.

Also, the subject method should be well tolerated based on the relative non-toxicity of anti-CD20 antibodies, such as RITUXAN®, and therefore should not adversely impact engraftment of the transplanted autologous cells. In fact, it may act to promote engraftment of such transplant.

As noted in the preferred embodiment, the purging agent will comprise RITUXAN®. However, other anti-CD20 antibodies may be used, e.g., other chimeric, primate, primatized®, humanized or human antibodies. Also, antibody fragments may be used, e.g., Fv's, FAB, F(ab)', F(ab₂)¹, and aggregates thereof.

In addition, antibodies and antibody fragments directed to other B cell surface markers, e.g., CD19, may also be used.

Methods for producing chimeric, primate, primatized®, humanized and human antibodies are well known in the art. See, e.g., U.S. Patent 5,530,101, issued to Queen et al, U.S. Patent 5,225,539, issued to Winter et al, U.S. Patents 4,816,397 and 4,816,567, issued to Boss et al, and Cabilly et al, respectively, all of which are incorporated by reference in their entirety.

The selection of human constant regions may be significant to the therapeutic efficacy of the subject anti-CD20 antibody. In the preferred embodiment, the subject anti-CD20 antibody will comprise human, gamma 1, or gamma 3 constant regions and, more preferably, human gamma 1 constant regions. The use of gamma 1 anti-CD20 antibodies as therapeutics is disclosed in U.S. Patent 5,500,362, issued to Robinson et al.

Methods for making human antibodies are also known and include, by way of example, production in SCID mice, and *in vitro* immunization.

As noted, a particularly preferred chimeric anti-CD20 antibody is RITUXAN®, which is a chimeric gamma 1 anti-human CD20 antibody. The complete amino acid and corresponding nucleic acid sequence for this antibody may be found in U.S. Patent 5,736,137. This antibody, which is produced in a proprietary CHO cell expression system commercialized by IDEC Pharmaceuticals Corporation, is made by a CHO cell transfectoma which was deposited on November 4, 1992, under the provisions of the Budapest Treaty at the American Type Culture Collection, located at 12301 Parklawn Drive, Rockville, MD 20852. This cell line was determined to be viable and will be replaced should it become non-viable during the term of deposit. This cell line was made irrevocably available upon issuance of the 5,736,137 patent and is available without restriction from the ATCC. This cell line will also be available without restriction during the lifetime of any patent that may issue based on this application.

The subject anti-CD20 antibody, when used as a purging agent, will be administered by various routes of administration, typically parenteral. This is intended to include intravenous, intramuscular, subcutaneous, rectal, vaginal, and administration with intravenous infusion being preferred.

The anti-CD20 antibody will be formulated for therapeutic usage by standard methods, e.g., by addition of pharmaceutically acceptable buffers, e.g., sterile saline, sterile buffered water, propylene glycol, and combinations thereof.

### EXAMPLE

A single-arm pivotal study of Rituximab infused at 375 mg/m² weekly times four was conducted in 166 patients with relapsed or refractory, low-grade or follicular NHL (International Working Formulation [IWF] Types A - D and REAL classification, small lymphocytic lymphoma, Follicular center, follicular Grades I, II, III). (McLaughlin P, Grillo-López A, Link B, Levy R, Czuczman M, Williams M, Heyman M, Bence-Bruckler I, White C, Cabanillas F, Jain V, Ho A, Lister J, Wey K, Shen D, Dallaire B. Rituximab® chimeric anti-CD20 monoclonal antibody therapy for relapsed indolent lymphoma: half of patients respond to a 4-dose treatment program. Journal of Clinical Oncology 1998; 16:2825-2833.) Patients with tumor masses > 10 cm or with > 5000 lymphocytes/µL in the peripheral blood were excluded from this study. The median age was 58 years (105 men and 61 women) and the median number of prior treatments was three. Bone marrow involvement was present in 56% of 149 patients evaluated. Forty-five percent had ≥ 2 extranodal sites and 41 % had bulky disease (≥ 5 cm).

Complete response required the regression of all lymph nodes to < 1 × 1 cm² demonstrated on two occasions at least 28 days apart on neck, chest, abdomen, and pelvic CT scans, resolution of all symptoms and signs of lymphoma, and normalization of bone marrow, liver, and spleen. Partial response required a ≥ 50% decrease in the sum of the products of perpendicular measurements of lesions without any evidence of progressive disease for at least 28 days. Patients who did not achieve a CR or PR were considered non-responders, even if a net decrease (> 50%) of measurable disease was observed. Time to progression was measured from the first infusion until progression.

The overall response rate (ORR) was 48% with a 6% CR and a 42% PR rate (McLaughlin P, Grillo-López A, Link B, Levy R, Czuczman M, Williams M, Heyman M, Bence-Bruckler I, White C, Cabanillas F, Jain V, Ho A, Lister J, Wey K, Shen D, Dallaire B. Rituximab chimeric anti-CD20 monoclonal antibody therapy for relapsed indolent lymphoma: half of patients respond to a 4-dose treatment program. Journal of Clinical Oncology 1998; 16:2825-2833). The median time to progression (TTP) for responders was 13.2 months and the median duration of response (DR) was 11.6 months. Twenty-two of 80 (28%) responders remain in ongoing remission at 20.9+ to 32.9+ months (McLaughlin P, Grillo-López A, Maloney D, Link B, Levy R, Czuczman M, Cabanillas F, Dallaire B, White C. Efficacy controls in long-term follow-up of patients treated with rituximab for relapsed or refractory, low-grade or follicular NHL. Blood 1998; 92:414a-415a).

Administration of Rituximab resulted in a rapid and sustained depletion of B-cells. Circulating B-cells were depleted within the first three doses with sustained depletion for up to six to nine months post-treatment in 83% of patients. Median B-cell levels returned to normal by 12 months following treatment. Although median NK cell counts remained unchanged, a positive correlation was observed between higher absolute NK cell counts at baseline and response to Rituximab (Janakiraman N, McLaughlin P, white C, Maloney D, Shen D, G-rillo-López A. Rituximab: Correlation between effector cells and clinical activity in NHL. Blood 1998; 92 (10 Suppl 1):337a).

Several baseline prognostic factors were analyzed to determine their correlation to response. Significantly, in 23 patients relapsed after ABMT or PBSC, the ORR was 78% versus 43% in patients who did not undergo prior high-dose therapy (p< 0.01). This suggests that anti-CD20 treatment may effectively be used to purge CD20 antigen-expressing cells *in vivo* when administered following transplantation. Moreover, because patients who receive prior high dose therapy accompanied a bone marrow or peripheral stem cell transplantation appear to benefit more from subsequent Rituximab therapy than those patients without prior therapy and transplantation, this suggests that a combined treatment protocol including bone marrow or stem cell transplantation provides a synergistic effect when compared to either single treatment alone.

Although the present invention has been described in some detail by way of illustration and example, for purposes of clarity and understanding it will be apparent that curtain changes and modifications may be practical within the scope of the appended claims.

## Claims

1. Use of an anti-CD20 antibody for the preparation of a composition for reducing the risk of relapse of a B-cell-related disease in a patient receiving a bone marrow or peripheral blood stem cell transplant, which patient has previously been treated under conditions designed to eradicate disease-causing B-cells, wherein the composition is for treating said transplant *in vivo* concurrent with or up to 100 days after transplantation with an amount of an anti-CD20 antibody effective to purge the number of disease-causing CD20 antigen-expressing cells in the patient.

2. Use according to claim 1, wherein said composition is for effecting purging *in vivo* by administering rituximab no later than about one month after transplant.

3. Use according to claim 1 or claim 2, wherein the composition comprises rituximab and is for administration at a dosage ranging from about 0.1 to 20 mg/kg at about one week after transplant.

4. Use according to claim 3, wherein said patient has previously been subjected to a treatment protocol selected from the group consisting of whole body irradiation, rituximab immunotherapy, chemotherapy, cytokine therapy, radioimmunotherapy, or a combination thereof.

5. Use according to claim 4, wherein the patient has previously been subjected to radioimmunotherapy.

6. Use according to any one of claims 1 to 5, wherein said disease is B-cell lymphoma or leukemia.

7. Use accordingly to claim 6, wherein said disease is non-Hodgkin's lymphoma.

8. A composition comprising an anti-CD20 antibody for use in a method of treatment for reducing the risk of relapse of a B-cell-related disease in a patient receiving a bone marrow or peripheral blood stem cell transplant, which patient has previously been treated under conditions designed to eradicate disease-causing B-cells, wherein the composition is for treating said transplant *in vivo* concurrent with or up to 100 days after transplantation with an amount of an anti-CD20 antibody effective to purge the number of disease-causing CD20 antigen-expressing cells in the patient.

9. The composition for use according to claim 8, wherein said composition is for effecting purging *in vivo* by administering rituximab no later than about one month after transplant.

10. The composition for use according to claim 8 or claim 9, wherein the composition comprises rituximab and is for administration at a dosage ranging from about 0.1 to 20 mg/kg at about one week after transplant.

11. The composition for use according to claim 10, wherein said patient has previously been subjected to a treatment protocol selected from the group consisting of whole body irradiation, rituximab immunotherapy, chemotherapy, cytokine therapy, radioimmunotherapy, or a combination thereof.

12. The composition for use according to claim 11, wherein the patient has previously been subjected to radioimmunotherapy.

13. The composition for use according to any one of claims 8 to 12, wherein said disease is B-cell lymphoma or leukemia.

14. The composition for use accordingly to claim 13, wherein said disease is non-Hodgkin's lymphoma.

## Patentansprüche

1. Verwendung eines Anti-CD20-Antikörpers zur Herstellung einer Zusammensetzung zur Reduktion des Risikos eines Rückfalls einer mit B-Zellen in Verbindung stehenden Erkrankung bei einem Patienten, der eine Knochenmarks- oder Periphere-Blutstammzellen-Transplantation erhält, wobei der Patient zuvor unter Bedingungen behandelt wurde, die so ausgewählt wurden, um die die Krankheit hervorrufenden B-Zellen abzutöten, worin die Zusammensetzung zur Behandlung des Transplantats in vivo gleichzeitig mit oder bis zu 100 Tage nach der Transplantation mit einer Menge an Anti-CD20-Antikörpern bestimmt ist, die wirksam ist, um die Anzahl der die Krankheit hervorrufenden CD20-Antigen-exprimierenden Zellen bei dem Patienten zu klären.

2. Verwendung nach Anspruch 1, worin die Zusammensetzung zur Ausführung der In-vivo-Klärung durch Verabreichung von Rituximab nicht später als etwa einen Monat nach der Transplantation dient.

3. Verwendung nach Anspruch 1 oder Anspruch 2, worin die Zusammensetzung Rituximab umfasst und zur Verabreichung in einer Dosierung, die von etwa 0,1 bis 20 mg/kg reicht, etwa eine Woche nach der Transplantation dient.

4. Verwendung nach Anspruch 3, worin der Patient zuvor einem Behandlungsprotokoll unterzogen wurde, das aus der aus einer Ganzkörperbestrahlung, Rituximab-Immuntherapie, Chemotherapie, Zytokintherapie, Radioimmuntherapie oder einer Kombination daraus bestehenden Gruppe ausgewählt wurde.

5. Verwendung nach Anspruch 4, worin der Patient zuvor einer Radioimmuntherapie unterzogen wurde.

6. Verwendung nach einem der Ansprüche 1 bis 5, worin die Erkrankung ein B-Zellen-Lymphom oder Leukämie ist.

7. Verwendung nach Anspruch 6, worin die Erkrankung ein Non-Hodgkin-Lymphom ist.

8. Zusammensetzung, umfassend einen Anti-CD20-Antikörper, zur Verwendung in einem Behandlungsverfahren zur Reduktion des Risikos eines Rückfalls einer mit B-Zellen in Verbindung stehenden Erkrankung bei einem Patienten, der eine Knochenmarks- oder Periphere-Blutstammzellen-Transplantation erhält, wobei der Patient zuvor unter Bedingungen behandelt wurde, die so ausgewählt wurden, um die die Krankheit hervorrufenden B-Zellen abzutöten, worin die Zusammensetzung zur Behandlung des Transplantats in vivo gleichzeitig mit oder bis zu 100 Tage nach der Transplantation mit einer Menge an Anti-CD20-Antikörpern bestimmt ist, die wirksam ist, um die Anzahl der die Krankheit hervorrufenden CD20-Antigen-exprimierenden Zellen bei dem Patienten zu klären.

9. Zusammensetzung zur Verwendung nach Anspruch 8, worin die Zusammensetzung zur Ausführung der In-vivo-Klärung durch Verabreichung von Rituximab nicht später als etwa einen Monat nach der Transplantation dient.

10. Zusammensetzung zur Verwendung nach Anspruch 8 oder Anspruch 9, worin die Zusammensetzung Rituximab umfasst und zur Verabreichung in einer Dosierung, die von etwa 0,1 bis 20 mg/kg reicht, etwa eine Woche nach der Transplantation dient.

11. Zusammensetzung zur Verwendung nach Anspruch 10, worin der Patient zuvor einem Behandlungsprotokoll unterzogen wurde, das aus der aus einer Ganzkörperbestrahlung, Rituximab-Immuntherapie, Chemotherapie, Zytokintherapie, Radioimmuntherapie oder einer Kombination daraus bestehenden Gruppe ausgewählt wurde.

12. Zusammensetzung zur Verwendung nach Anspruch 11, worin der Patient zuvor einer Radioimmuntherapie unterzogen wurde.

13. Zusammensetzung zur Verwendung nach einem der Ansprüche 8 bis 12, worin die Erkrankung ein B-Zellen-Lymphom oder Leukämie ist.

14. Zusammensetzung zur Verwendung nach Anspruch 13, worin die Erkrankung ein Non-Hodgkin-Lymphom ist.

## Revendications

1. Utilisation d'un anticorps anti-CD20 pour la préparation d'une composition pour réduire le risque d'une rechute d'une maladie liée à la cellule B dans un patient recevant une transplantation de moelle osseuse ou de cellules souches de sang périphérique, ledit patient ayant été traité préalablement sous des conditions conçues pour éradiquer les cellules B provoquant la maladie, où la composition est pour le traitement de ladite greffe in vivo conjointement avec ou jusqu'à 100 jours après la transplantation avec une quantité d'anticorps anti-CD20 efficaces pour purger le nombre de cellules d'expression d'antigène CD20 provoquant la maladie dans le patient.

2. Utilisation selon la revendication 1, dans laquelle ladite composition est pour effectuer une purge in vivo en administrant du rituximab pas plus tard qu'environ un mois après la greffe.

3. Utilisation selon la revendication 1 ou la revendication 2, dans laquelle la composition comprend du rituximab et est pour l'administration à un dosage d'environ 0,1 à 20 mg/kg à environ une semaine après la greffe.

4. Utilisation selon la revendication 3, dans laquelle ledit patient a été soumis préalablement à un protocole de traitement sélectionné dans le groupe consistant en irradiation totale, immunothérapie par rituximab, chimiothérapie, thérapie à base de cytokines, radioimmunothérapie ou une combinaison de ceux-ci.

5. Utilisation selon la revendication 4, dans laquelle le patient a été soumis préalablement à une radioimmunothérapie.

6. Utilisation selon l'une quelconque des revendications 1 à 5, dans laquelle ladite maladie est un lymphome des cellules B ou une leucémie.

7. Utilisation selon la revendication 6, dans laquelle ladite maladie est un lymphome non hodgkinien.

8. Composition comprenant un anticorps anti-CD20 pour utilisation dans une méthode de traitement pour réduire le risque d'une rechute d'une maladie liée aux cellules B dans un patient recevant une greffe de moelle osseuse ou de cellules souches de sang périphérique, ledit patient ayant été traité préalablement sous des conditions conçues pour éradiquer les cellules B provoquant la maladie, où la composition est pour le traitement de ladite greffe in vivo conjointement avec ou jusqu'à 100 jours après la greffe avec une quantité d'anticorps anti-CD20 efficace pour purger le nombre de cellules d'expression d'antigène de CD20 provoquant la maladie dans le patient.

9. Composition pour utilisation selon la revendication 8, où ladite composition est pour effectuer la purge in vivo en administrant du rituximab pas plus tard qu'environ un mois après la greffe.

10. Composition pour utilisation selon la revendication 8 ou la revendication 9, où la composition comprend du rituximab et est pour l'administration à un dosage d'environ 0,1 à 20 mg/kg environ une semaine après la greffe.

11. Composition pour utilisation selon la revendication 10, où ledit patient a été soumis préalablement à un protocole de traitement sélectionné dans le groupe consistant en irradiation totale, immunothérapie par rituximab, chimiothérapie, thérapie à base de cytokines, radio immunothérapie ou une combinaison de ceux-ci.

12. Composition pour utilisation selon la revendication 11, dans laquelle le patient a été soumis préalablement à une radioimmunothérapie.

13. Composition pour utilisation selon l'une quelconque des revendications 8 à 12, où ladite maladie est un lymphome des cellules B ou une leucémie.

14. Composition pour utilisation selon la revendication 13, où ladite maladie est un lymphome non hodgkinien.
